# Europäisches Patentamt

## European Patent Office
## Office européen des brevets

(19)

(11) Veröffentlichungsnummer: **0 154 829 B1**

(12) **EUROPÄISCHE PATENTSCHRIFT**

(45) Veröffentlichungstag der Patentschrift:
**07.01.87**

(21) Anmeldenummer: **85101610.5**

(22) Anmeldetag: **18.03.83**

(60) Veröffentlichungsnummer der früheren Anmeldung
nach Art. 76 EPÜ: **0090283**

(51) Int. Cl.⁴: **C 07 C 11/02,** C 07 C 1/20,
B 01 J 29/04

(54) Verfahren zur Herstellung von Olefinen aus Methanol/Dimethyleter.

(30) Priorität: **27.03.82 DE 3211399**
**14.01.83 DE 3300982**

(43) Veröffentlichungstag der Anmeldung:
**18.09.85 Patentblatt 85/38**

(45) Bekanntmachung des Hinweises auf die Patenterteilung:
**07.01.87 Patentblatt 87/2**

(84) Benannte Vertragsstaaten:
**BE DE FR GB NL**

(56) Entgegenhaltungen:
**EP - A - 0 007 081**
**US - A - 4 292 458**

(73) Patentinhaber: **BASF Aktiengesellschaft,**
**Carl-Bosch-Strasse 38, D-6700 Ludwigshafen (DE)**

(72) Erfinder: **Hoelderich, Wolfgang, Dr., Mannheimer**
**Strasse 18 C, D-6710 Frankenthal (DE)**
Erfinder: **Mross, Wolf Dieter, Dr.,**
**Anselm-feurbach-Strasse 21, D-6710 Frankenthal (DE)**
Erfinder: **Schwarzmann, Matthias, Dr.,**
**Carl-Bosch-Strasse 54, D-6703 Limburgerhof (DE)**

EP 0 154 829 B1

## Beschreibung

In neuerer Zeit gewinnen Bemühungen, Methanol zur Herstellung von Olefinen zu verwenden, zunehmendes Interesse. Methanol lässt sich aus Kohle, über Kohlevergasung und Herstellung von Synthesegas, mit Hilfe bewährter Technologien leicht herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff beibehalten werden. In den vergangenen Jahren sind daher Verfahren entwickelt worden, die die Herstellung von Olefinen aus Methanol und/oder Dimethylether zum Gegenstand haben.

Ein solches Verfahren ist beispielsweise in der DE-OS 26 15 150 beschrieben. Als Katalysator wird ein Aluminosilikatzeolith ZSM-5 verwendet, der eigentlich ein Aromatisierungskatalysator ist. Die Umsetzung kann aber durch verschiedene Massnahmen, insbesondere durch die Verkürzung der Verweilzeit, in Richtung Olefinbildung gelenkt werden. Weitere die Olefinbildung begünstigende Parameter sind insbesondere die Verdünnung von Methanol bzw. Dimethylether mit Inertgasen bzw. Wasserdampf. Die Erfahrung zeigt, dass hohe Olefinausbeuten nur durch eine sehr starke Verdünnung von Methanol und/oder Dimethylether mit Inertgas oder Wasserdampf zu erzielen sind. Andere bekannte Verfahren haben als Nachteil eine geringe Belastbarkeit und eine schnelle Verkokung des Katalysators. Die Verdünnung des Katalysators mit Bindemittel soll ebenfalls ein für die Olefinbildung vorteilhafte Massnahme sein, doch werden durch die bisher genutzten Binder auf Basis von Aluminiumoxiden Nebenreaktionen und auch Desaktivierung des Katalysators verursacht.

Es wurde nun gefunden, dass man $C_2$- bis $C_4$-Olefine in hoher Ausbeute aus Methanol und/oder Dimethylether durch katalytische Umsetzung bei erhöhter Temperatur in Gegenwart von Borosilikatzeolithen erhält, wenn man als Katalysatoren Borosilikatzeolithe verwendet, die mit Bindemittelgemischen, aus Siliziumdioxid und Aluminiumoxid, deren Aluminiumoxidgehalt $\leqslant 10$ Gew.-% ist, verformt sind.

Zweckmässig verwendet man für die Verformung z.B. Bindemittelgemische aus hochdispersem Siliciumdioxid und hochdispersem Aluminiumoxid, oder Bindemittelgemische aus hochdispersem Siliciumdioxid und Boehmit oder Bindemittelgemische aus Kieselgel und Boehmit.

Vorteilhaft verwendet man Katalysatoren, bei denen die Borosilikatzeolithe mit hochdispersem Bindemittel, das aus 90-98 Gew.-% hochdispersem $SiO_2$ und 10-2 Gew.-% hochdispersem $Al_2O_3$ besteht, verformt werden. Als weitere vorteilhafte Binder eignen sich Kieselgel und/oder pyrogene Kieselsäure gemischt mit Boehmit ($\leqslant 10$ Gew.-%). Unter hochdispersen $SiO_2$ und $Al_2O_3$ versteht man Oxide, die aus den entsprechenden Halogeniden durch Pyrolyse hergestellt worden sind. Die Oberflächen von $SiO_2$ gemessen durch Brunner, Emmert, Teller in qm²/g betragen 130 bis 380 und

die Oberflächen von $Al_2O_3$ 100 bis 200 qm²/g und das Gemisch aus $SiO_2$ und $Al_2O_3$ 80 bis 170 qm²/g.

Erfindungswesentlich ist, dass der Katalysator einen Restaluminiumoxidgehalt aufweisen muss; die Anwesenheit von Aluminiumoxid ist besonders vorteilhaft bei der Durchführung der Umsetzung von Methanol in einem Rohrreaktor unter isothermen Bedingungen, um die erste Stufe der Reaktion — die Dehydratation des Methanols zu Dimethylether — einzuleiten. Der mit Aluminiumoxid verstrangte Bor-Zeolith ist quasi ein bifunktioneller Katalysator; das Aluminiumoxid dient zur Dehydratation des Methanols zu Dimethylether und der Bor-Zeolith setzt diesen um zu den gewünschten Olefinen. Ausschliesslich mit Kieselgel oder hochdispersen Kieselsäuren verstrangte Borosilikatzeolithe erfordern für die Reaktion mit Methanol Temperaturen oberhalb 550° C bzw. die Zufuhr oder die Rückvermischung von Olefinen bzw. der Einsatz von reinem Dimethylether.

Zur Herstellung der Katalysatoren werden die Borsilikatzeolithe mit den jeweiligen Bindemitteln bzw. Bindemittelgemischen zusammen zu Tabletten oder Strängen verformt.

Bei der Durchführung des Verfahrens wird Methanol, das mit Dimethylether im Gleichgewicht steht, bei einem Druck zwischen Normaldruck und etwa 30 bar, vorzugsweise bei 0 bis 1 bar und bei Temperaturen zwischen 300° C und 650° C, vorzugsweise bei 400° C bis 550° C, an den oben beschriebenen Katalysatoren umgesetzt. Das Methanol kann einen Wassergehalt bis zu 90 Gew.-% haben, zweckmässig verwendet man als Ausgangsstoff Rohmethanol, das etwa 20% Wasser enthält.

Dem Methanol können auch noch andere niedere Alkohole beigemischt sein. Die Belastung des Katalysators, ausgedrückt in WHSV = $h^{-1}$ — g Methanol pro g Katalysator und Stunde —, wird zweckmässig so gewählt, dass die Ausgangsstoffe möglichst quantitativ umgesetzt werden, so dass keine Abtrenn- und Rückführprobleme für nicht umgesetzten Methanol/Dimethylether entstehen. Im allgemeinen soll daher die WHSV im Bereich von 0,5 bis 50 $h^{-1}$, vorzugsweise im Bereich von 2 bis 15 $h^{-1}$ liegen.

Durch das erfindungsgemässe Verfahren wird die Olefinselektivität im $C_2$- bis $C_4$-Bereich bei der Methanolumwandlung zu Kohlenwasserstoffen, insbesondere im Temperaturbereich von 400° C bis 600° C wesentlich erhöht.

Die Bildung unerwünschter Nebenprodukte wie Methanol und Aromaten, die Aromaten bei Verwendung von Aluminiumoxidbindern entstehen, werden durch den erfindungsgemässen Katalysator stark zurückgedrängt. Diese Tatsache äussert sich vorteilhaft in der Erhöhung der Laufzeit des eingesetzten Katalysators.

Unter Laufzeit versteht man die Zeit zwischen zwei Regenerierungen. Auch die Lebensdauer des Katalysators wird insgesamt verlängert. Der erfindungsgemässe Effekt der Laufzeitverbesserung wirkt sich besonders bei der Umsetzung bei hohen Temperaturen, z.B. im Bereich von 450°C bis 550°C, aus.

Es ist ein weiterer Vorteil der Erfindung, dass man die Umsetzung zu $C_2$- bis $C_4$-Olefinen mit Rohmethanol ohne weiteren Zusatz von inerten Verdünnungsmitteln wie $N_2$, He oder $H_2O$ ausführen kann.

Die Durchführung des erfindungsgemässen Verfahrens wird anhand der nachstehenden Beispiele näher erläutert.

*Beispiel*

*Katalysator A*

Der Bor-Zeolith wird in einer hydrothermalen Synthese aus 64 g $SiO_2$ (pyrogene Kieselsäure), 12,2 g $H_3BO_3$, 800 g einer wässerigen 1,6-Hexandiamin-Lösung (Mischung 50:50) bei 170°C unter autogenem Druck in einem Rührautoklaven hergestellt. Nach Abfiltrieren und Auswaschen wird das kristalline Reaktionsprodukt bei 110°C/24 h getrocknet und bei 500°C/24 h calciniert. Dieser Borosilikatzeolith setzt sich zusammen aus 94,2 Gew.-% $SiO_2$ und 2,32 Gew.-% $B_2O_3$.

60 g dieses Borosilikatzeolithen werden mit 36,5 g hochdispersem $SiO_2$ und 3,5 g hochdispersem $Al_2O_3$ zu 2 mm-Strängen bei einem Pressdruck von 70 bar verformt. Das Extrudat wird bei 110°C/16 h getrocknet und bei 500°C/16 h calciniert.

*Katalysator B*

Katalysator B wird wie Katalysator A erhalten, lediglich als Bindemittel werden 30 g hochdisperses $SiO_2$ (Aerosil) und 10 g Boehmit eingesetzt.

*Katalysator C*

Die Herstellungsweise von Katalysator C entspricht der von den Katalysatoren A und B, jedoch werden hierbei 30 g Kieselgel und 10 g Boehmit als Binder verwendet.

An diesen Katalysatoren A, B, C werden unter isothermen Bedingungen in einem Rohrreaktor Rohmethanol mit 20 Gew.-% Wasser bei 550°C und WHSV = 7,8 h$^{-1}$ bez. auf eingesetztes $CH_3OH$ quantitativ umgesetzt. Die Ausbeuten, bezogen auf eingesetztes $CH_2$, sind in der Tabelle — die Spalten A, B, C — angegeben.

Zum Ausbeutevergleich wurde folgender Katalysator herangezogen, der unter denselben Reaktionsbedingungen wie die Katalysatoren A, B, C getestet wurde.

*Vergleichskatalysator D*

Katalysator D wird erhalten durch Verstrangen des oben beschriebenen Borosilikatzeolithen mit Boehmit im Verhältnis 60:40. Die Trocknung erfolgt bei 100°C/16 h und die Calcinierung bei 500°C/16 h.

| Katalysator | A | B | C | D |
|---|---|---|---|---|
| $C_2H_4$ | 11.3 | 10.7 | 7.4 | 12.0 |
| $C_3H_6$ | 42.6 | 39.8 | 39.5 | 32.9 |
| $C_4H_8$ | 23.1 | 20.7 | 20.1 | 16.1 |
| $CH_4$ | 1.5 | 3.6 | 2.6 | 7.9 |
| $C_2H_6$ | 0.3 | 0.6 | 0.3 | 0.6 |
| $C_3H_8$ | 1.7 | 1.9 | 1.8 | 2.1 |
| $C_4H_{10}$ | 1.1 | 1.3 | 1.2 | 1.2 |
| $C_5^+$-Aliphate | 12.2 | 10.5 | 15.0 | 9.4 |
| $C_6^+$-Aromaten | 4.1 | 8.9 | 10.2 | 15.9 |
| Laufzeit h | 15 | 10 | 10 | 7 |
| g $CH_3OH$/g Katalysator | 117 | 78 | 78 | 55 |

**Patentansprüche**

1. Verfahren zur Herstellung von Olefinen durch katalytische Umsetzung von Methanol und/ oder Dimethylether in Gegenwart von Borosilikatzeolithen bei erhöhter Temperatur, dadurch gekennzeichnet, dass man als Katalysatoren Borosilikatzeolithe verwendet, die mit Bindemittelgemischen aus Siliziumdioxid und Aluminiumoxid, deren Aluminiumoxidgehalt $\leqslant$ 10 Gew.-% ist, verformt sind.

2. Verfahren nach Anspruch 1, dadurch gekennzeichnet, dass man für die Verformung Bindemittelgemische aus hochdispersem Siliziumdioxid und hochdispersem Aluminiumoxid verwendet.

3. Verfahren nach Anspruch 1, dadurch gekennzeichnet, dass man für die Verformung Bindemittelgemische aus hochdispersem Siliziumdioxid und Böhmit verwendet.

4. Verfahren nach Anspruch 1, dadurch gekennzeichnet, dass man für die Verformung Bindemittelgemische aus Kieselgel und Böhmit verwendet.

## Claims

1. A process for the preparation of olefins by catalytic reaction of methanol and/or dimethyl ether in the presence of a borosilicate zeolite at elevated temperature, wherein the catalyst used is a borosilicate zeolite which has been molded together with a binder mixture of aluminum oxide and silicon dioxide, the aluminum oxide content being ⩽ 10% by weight.

2. A process as claimed in claim 1, wherein a binder mixture of highly dispersed silicon dioxide and highly dispersed aluminum oxide is used for molding.

3. A process as claimed in claim 1, wherein a binder mixture of highly dispersed silicon dioxide and boehmite is used for molding.

4. A process as claimed in claim 1, wherein a binder mixture of silica gel and boehmite is used for molding.

## Revendications

1. Procédé de préparation d'oléfines par réaction catalytique de méthanol et/ou éther diméthylique en présence de zéolithes de borosilicate, à température élevée, caractérisé par le fait que l'on utilise, comme catalyseurs, des zéolithes de borosilicate qui sont mis en forme par des mélanges-liants de bioxyde de silicium et oxyde d'aluminium, dont la teneur en oxyde d'aluminium est ⩽ 10% en poids.

2. Procédé selon la revendication 1, caractérisé par le fait que l'on utilise, pour la mise en forme, des mélanges-liants de bioxyde de silicium fortement dispersé et d'oxyde d'aluminium fortement dispersé.

3. Procédé selon la revendication 1, caractérisé par le fait que l'on utilise, pour la mise en forme, des mélanges-liants de bioxyde de silicium fortement dispersé et de bœhmite.

4. Procédé selon la revendication 1, caractérisé par le fait que l'on utilise, pour la mise en forme, des mélanges-liants de gel de silice et de bœhmite.